# EUROPÄISCHE PATENTSCHRIFT

(11) **EP 0 501 207 B1**
(45) Veröffentlichungstag und Bekanntmachung des Hinweises auf die Patenterteilung: **22.12.1993**
(21) Anmeldenummer: 92102072.3
(22) Anmeldetag: 07.02.1992
(51) Int. Cl.: A61F 2/34, A61F 2/40, A61B 17/58

(54) **Gelenkpfanne**
Acetabular cup
Coque acétabulaire

(30) Priorität: 28.02.1991 DE 4106272
(43) Veröffentlichungstag der Anmeldung: 02.09.1992
(73) Patentinhaber: CHIROPRO GmbH, 90766 Fürth (DE)
(72) Erfinder: Eulert, Jochen, Prof. Dr. med., Orthopäd. Klinik, W-8700 Würzburg (DE); Worsch, Horst, W-8501 Rothenberg (DE)
(74) Vertreter: Gaiser, Hartmut, Dipl.-Ing.

(56) Entgegenhaltungen:
- EP-A- 0 123 514
- EP-A- 0 242 719
- EP-A- 0 295 912
- EP-A- 0 402 810
- FR-A- 2 578 162

## Beschreibung

Die Erfindung betrifft eine Gelenkpfanne gemäß dem Oberbegriff des Anspruchs 1, wie sie aus der EP-A-0 123 514 bekannt ist.

Eine weitere Gelenkpfanne ist in der DE 36 29 799 C2 beschrieben. Eine derartige Endoprothese soll schnell und einfach implantiert werden können.

Nach der DE 36 29 799 C2 sind außen am Grundkörper zwei Ansatze angeformt. Jeder der Ansätze weist eine Aufnahmebohrung auf. In die Aufnahmebohrungen sind Zapfen von Anschlußelementen einsteckbar. Die Anschlußelemente sind mit Löchern zur Schraubbefestigung an einem Knochenteil versehen. In der DE 36 29 799 C2 ist betont, daß die Anschlußelemente das Ausrichten des Grundkörpers bei der Implantation der Prothese erleichtern. Es ist dabei vorausgesetzt, daß die Anschlußelemente an den Ansätzen festgelegt werden, bevor der Grundkörper implantiert wird. Eine umgekehrte Vorgehensweise ist kaum möglich, da die Anschlußelemente sich nach der Implantation des Grundkörpers höchstens in Sonderfällen in dessen Ansätze einstecken lassen. Die Ansätze überragen den Grundkörper außen weit, so daß sich der Grundkörper nicht zum Einsetzen in eine teilkugelförmige vorbereitete Stelle eines Knochens eignet.

In der EP 0 242 719 Bl ist eine Hüftgelenkspfanne beschrieben, die einen rotationssymmetrischen Grundkörper hat, welcher in eine teilkugelförmig vorbereitete Stelle des Knochens einsetzbar ist. Der Grundkörper besteht aus Kunststoff. An seinen um seine Höhlung bzw. seine Pfannenachse umlaufenden Rand sind mehrere nach außen radial vorstehende Laschen angeformt. Die Laschen sind zur Verbindung mit dem Beckenknochen vorgesehen. Hierfür konnen Schrauben verwendet werden. Nicht benötigte Laschen sollen während der Operation abgetrennt werden. Ein solcher Aufbau ist problematisch, da er sich widersprechende Bedingungen erfüllen muß. Einerseits sollen die Laschen möglichst fest mit dem Pfannenkörper verbunden sein und andererseits sollen sie leicht abtrennbar sein. Das Abtrennen der Laschen während der Implantation der Hüftgelenkspfanne ist problematisch, da zum Abtrennen ein Werkzeugeinsatz notwendig ist und bei einem solchen Werkzeugeinsatz der Knochen geschädigt werden kann.

In der DE 35 33 432 Al ist eine Hüftpfanne mit halbkugelähnlicher Raumform zur Implantation in einen Hüftknochen beschrieben. Außen am Rand des Grundkörpers ist eine bestimmte Anzahl von Verankerungszapfen vorgesehen. All diese Verankerungszapfen müssen bei der Implantation der Hüftgelenkpfanne in den Knochen eingetrieben werden. Variationsmöglichkeiten bestehen nicht.

In der EP 0 341 199 Al ist eine Hüftgelenkspfanne beschrieben. Diese weist eine im Knochen mittels eines Außengewindes festzulegende Außenschale und einen in diese einsetzbaren Pfannenkörper auf. Der Pfannenkörper, nicht jedoch die Außenschale, ist an seinem Rand mit Bohrungen versehen. Diese Bohrungen dienen lediglich dem Eingriff eines Setzinstruments.

Nach der EP 0 295 912 Al sind am stirnseitigen Rand einer Hüftgelenkspfanne Bohrungen zum Einsetzen von Knochenschrauben vorgesehen. Mittels der Schrauben soll die Pfanne an dem Knochen befestigt werden. Die Bohrungen dienen nicht der Befestigung eines Bauteils an der Pfanne. Außerdem ist an dem stirnseitigen Rand der Hüftgelenkspfanne ein einzelner Zapfen angeordnet. Dieser dient der Ausrichtung eines Rings einer mit einer Vielzahl von Öffnungen versehenen Laschenplatte und der Ausrichtung eines in der Pfanne festlegbaren Kopfstückes.

In der EP 0 123 514 Al ist eine Hüftgelenkspfanne gezeigt, an der ein Ring anschraubbar ist, welcher ein Kopfstück an der Hüftgelenkspfanne hält. Außerdem ist an der Hüftgelenkspfanne einstückig eine Lasche ausgebildet, die am Knochen festschraubbar ist.

Aufgabe der Erfindung ist es, eine Gelenkpfanne der eingangs genannten Art vorzuschlagen, an deren Grundkörper sich auch nach dessen Implantation noch wenigstens ein Bauteil festlegen läßt und zwar in je nach der Notwendigkeit verschiedenen Positionen.

Erfindungsgemäß ist obige Aufgabe bei einer Gelenkpfanne der eingangs genannten Art durch die Merkmale des Kennzeichnenden Teils des Anspruchs 1 gelöst.

Bei dieser Gelenkpfanne läßt sich der Grundkörper im vorbereiteten Knochen implantieren, ohne daß darauf Rücksicht zu nehmen ist, in welchen Stellungen das Bauteil bzw. die Bauteile später zu positionieren sind. Durch die Vielzahl der am Rand verteilten gleichen Aufnahmelöcher kann bzw. können das Bauteil bzw. die Bauteile nach der Implantation des Grundkörpers an diesem in der im Einzelfall notwendigen Position angebracht werden. Dies erleichtert die Operation wesentlich.

Günstig ist auch, daß die Aufnahmelöcher die Implantation des Grundkörpers nicht stören und auch keine besondere Vorbereitung des Knochens notwendig machen. Die nicht benutzten Aufnahmelöcher oder auch alle Aufnahmelöcher, wenn kein Bauteil am Grundkörper festgelegt werden soll, bleiben frei. Die Gelenkpfanne ist aus den genannten Gründen vielseitig verwendbar. Dies bedeutet eine Kostenersparnis bei der Herstellung und eine Vereinfachung der Lagerhaltung.

Günstig ist auch, daß die Bauteile und der Grundkörper nicht aus dem gleichen Material bestehen müssen. So ist es beispielsweise möglich, den Grundkörper aus Metall und das Bauteil aus Metall oder Kunststoff, oder einem nach der Operation im Körper resorbierbaren Material herzustellen. Auch der Grundkörper kann aus Kunststoff bestehen.

Das Bauteil kann eine Lasche sein, die mit dem Knochen in der Umgebung des Grundkörpers verbindbar ist. Das Bauteil kann jedoch auch für andere Zwecke vorgesehen sein. Beispielsweise kann das Bauteil ein die Luxation eines in den Grundkörper bzw. dessen Einsatz eingesetzten künstlichen Gelenkkopf hemmendes Ringsegment sein, das die Innenform des Grundkörpers bzw. dessen Einsatzes so fortsetzt, daß der Halt des Gelenkkopfes unterstützt ist.

Die Gelenkpfanne eignet sich beispielsweise als Endoprothese für ein Hüftgelenk oder ein Schultergelenk.

Die Höhlung des Grundkörpers kann teilkreisförmig oder auch oval oder mehreckig sein.

Vorteilhafte Ausgestaltungen der Erfindung ergeben sich aus den Unteransprüchen und der folgenden Beschreibung eines Ausführungsbeispiels. In der Zeichnung zeigen:
Figur 1 einen Grundkörper einer Gelenkpfanne schematisch im Schnitt, beim Einsetzen einer Lasche,
Figur 2 eine Ansicht eines Grundkörpers der Gelenkpfanne in Richtung des Pfeiles II nach Figur 1,
Figur 3 einen Grundkörper bestückt mit mehreren Laschen,
Figur 4 einen Grundkörper mit im Vergleich zu Figur 3 anderen Laschen bestückt und
Figur 5 die Gelenkpfanne mit Einsatz im Schnitt, gegenüber Figur 1 verkleinert.

Eine Gelenkpfanne weist einen Grundkörper(1) auf. Dieser bildet eine etwa kugelförmige Halbschale mit einer Mittelachse(A). Im Grundkörper(1) besteht eine Höhlung(2) zur Aufnahme eines weiter unten beschriebenen Einsatzes.

Der Grundkörper(1) weist einen um die Mittelachse(A) umlaufenden Rand(3) auf, der eine ringförmige Stirnfläche(4) bildet, welche etwa in der äquatorialen Ebene der Kugelform liegt.

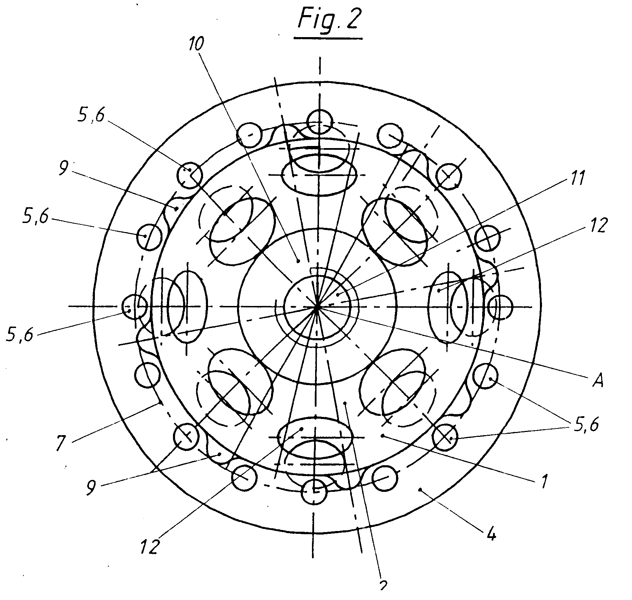

Im Rand(3) des Grundkörpers(1) sind Aufnahmelöcher(5) vorgesehen. Diese erstrecken sich von in der Stirnfläche(4) liegenden Öffnungen(6) in den Rand(3) hinein. Die Aufnahmmelöcher(5) weisen Längsachsen(L) auf, die parallel zur Mittelachse(A) liegen. Im Rand(3) ist eine Vielzahl solcher Aufnahmelöcher(5) vorgesehen. Im Beispielsfalle sind 16 Aufnahmelöcher(5) vorgesehen. Diese sind über den Umfang von 360° an der Stirnfläche(4) gleich verteilt, weisen also jeweils einen Winkelabstand von 22,5° auf (vgl. Figur 2).

Die Aufnahmelöcher(5) weisen im Ausführungsbeispiel einen senkrecht zur Längsachse(L) kreisförmigen Querschnitt auf. Dieser Querschnitt könnte jedoch auch anders, beispielsweise mehreckig, gestaltet sein. In ihn eingesteckte, entsprechend geformte Zapfen wären dann um die Längsachse(L) nicht drehbar, also verdrehgesichert.

Alle Aufnahmelöcher(5) schneiden sich mit einer Ringnut(7), die um die Mittelachse(A) im Grundkörper(1) umläuft und zur Höhlung(2) hin offen ist. In die Ringnut(7) ist ein radial geschlitzter Federring(8) eingesetzt.

Am Rand(3) des Grundkörpers(1) sind jeweils zwischen zwei Aufnahmelöchern(5) Aussparungen(9) vorgesehen. Diese sind an der Stirnfläche(4) und zur Höhlung(2) hin offen. Sie dienen der Drehsicherung des unten näher beschrieben Einsatzes um die Mittelachse(A).

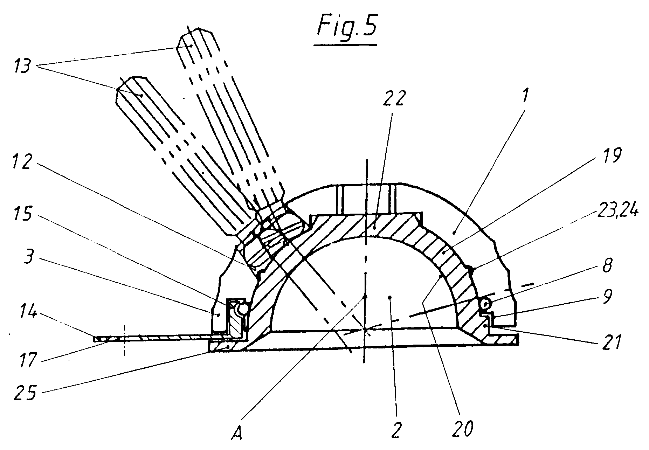

Der Grundkörper(1) weist in der Höhlung(2) eine zur Mittelachse(A) zentrale Vertiefung(10) mit anschließender Durchbrechung(11) für den unten genannten Einsatz auf. Außerdem ist der Grundkörper(1) mit einer Mehrzahl von Bohrungen(12) versehen, die sich zur Aufnahme von Befestigungsschrauben(13) eignen, die in den vorbereiteten Knochen des Patienten einzuschrauben sind und dann den Grundkörper(1) dort halten (vgl. Figuren 2 bis 5).

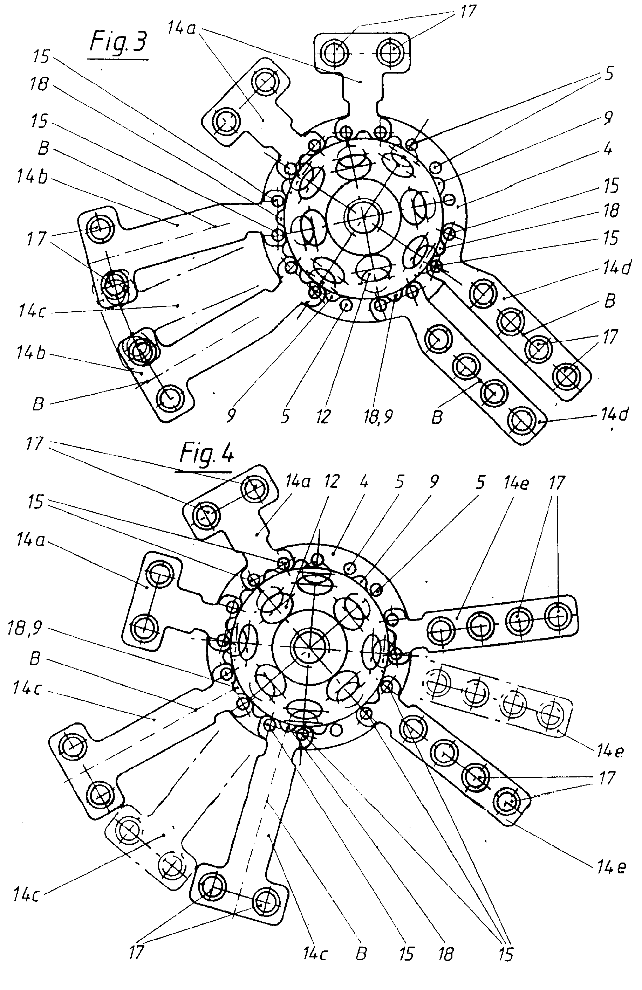

Zum Festlegen in den Aufnahmelöchern(5) sind bei den Ausführungsbeispielen nach den Figuren 3 bis 5 Laschen(14) als separate und verschieden gestaltete Bauteile vorgesehen. An jeder Lasche(14) sind zwei Zapfen(15) befestigt, deren Abstand dem Abstand der Aufnahmelöcher(5) gleich ist. Jede Lasche(14) läßt sich somit wahlweise mit ihren Zapfen(15) in zwei benachbarte Aufnahmelöcher(5) einstecken. Die Zapfen(15) weisen im Ausführungsbeispiel entsprechend den Aufnahmelöchern(5) kreisförmigen Querschnitt um die Längsachse(L) auf.

Durch die zwei Zapfen(15) jeder Lasche(14) ist erreicht, daß die Lasche(14) nach dem Einstecken in die Aufnahmelöcher(5) in der Ebene der Stirnfläche(4) nicht mehr verschwenkbar ist. Ist eine solche Verschwenkbarkeit erwünscht, dann wäre diese dadurch zu erreichen, daß an der Lasche(14) nur ein Zapfen(15) vorgesehen ist. Andererseits könnte auch mit nur einem Zapfen(15) eine Verschwenkbarkeit vermieden sein, wenn die Aufnahmelöcher(5) und die Zapfen(15) einen bezogen auf die Längsachse(L) von der Kreisform abweichenden Querschnitt, beispielsweise mehreckigen Querschnitt, aufwiesen.

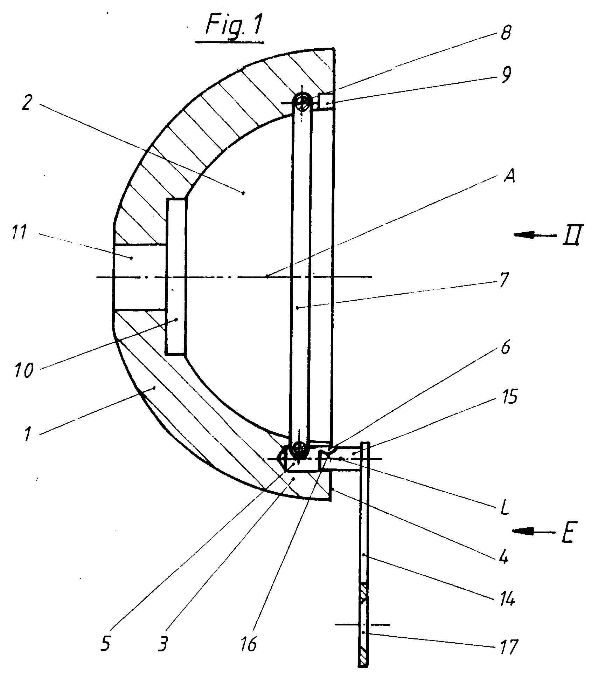

Die Zapfen(15) weisen eine Einbuchtung(16) auf, die dem Federring(8) angepaßt ist (vgl. Figur 1). Wird die Lasche(14) mit ihren Zapfen(15) in Richtung des Pfeiles(E) (vgl. Figur 1) in die betreffenden Aufnahmellöcher(5) gesteckt, dann schnappt der Federring(8) in die Einbuchtungen(16) der Zapfen(15). Es liegt dann die Lasche(14) auf der Stirnfläche(4) auf. Die Lasche(14) erstreckt sich dabei in der zur Mittelachse(A) senkrechten Ebene nach außen über den Grundkörper(1) hinaus. Sie kann - wenn sie entsprechend geformt ist - sich auch in einer zur Mittelachse(A) schrägen Ebene erstrecken.

Die Laschen(14) sind mit einer oder mehreren Bohrungen(17) versehen. Diese Bohrungen(17) sind an den Laschen(14) so angeordnet, daß die Lasche(14) am Knochen in der Umgebung des Grundkörpers(1) angeschraubt werden kann. Die Laschen(14) weisen zwischen den Zapfen(15) Ausschnitte(18) auf, die die Aussparungen(9) des Grundkörpers(1) nicht verdecken, sondern freilassen.

In den Figuren 3 und 4 sind Laschen(14) in unterschiedlichen Gestaltungen und in verschiedenen Anordnungen am Grundkörper(1) als Übersicht dargestellt. Es ist nicht erforderlich, daß in einem speziellen Einsatzfall gerade die in den Figuren 3 und 4 dargestellte Laschenanordnung gewählt wird. Eine oder mehrere der Laschen(14) lassen sich im jeweiligen Einsatzfall nach den Gegebenheiten verwenden.

In den Figuren 3 und 4 sind kurze Laschen(14a) gezeigt, die zwei Bohrungen(17) aufweisen. In Figur 3 sind außerdem zwei lange Laschen(14b) gezeigt, die ebenfalls zwei Bohrungen(17) aufweisen und die gegensinnig so abgewinkelt sind, daß ihre Längsachsen(B) in spitzen Winkeln zur Radialrichtung der Mittelasche(A) stehen.

Strichpunktiert ist zwischen den Laschen(14b) die Positionierung einer Lasche(14c) angedeutet, die dann angeordnet werden könnte, wenn die Laschen(14b) nicht vorgesehen sind. Außerdem sind in Figur 3 weitere Laschen(14d) gezeigt, die in Richtung ihrer Längsachse(B) mit Bohrungen(17) versehen sind und deren Längsachsen(B) gegensinnig in spitzen Winkeln zur Radialrichtung der Mittelachse(A) verlaufen.

In Figur 4 sind Laschen(14a,14c,14e) gezeigt, deren Längsachsen(B) radial zur Mittelachse(A) verlaufen. Die Laschen(14a) und die Laschen(14c) weisen jeweils zwei zur Mittelachse(A) umfängliche Bohrungen(17) auf. Die Laschen(14e) zeigen, wie die Laschen(14d) in Richtung der Längsachse(B) liegende Bohrungen(17). Die strichpunktierten Laschen(14c,14e) können dann gesetzt werden, wenn die anderen Laschen(14c,14e) nicht eingesetzt sind. Aus den Figuren 3 und 4 ist ersichtlich, daß mit verschiedenen Laschentypen je nach dem Bedarfsfall ein großer Bereich um den Grundkörper(1) herum zu dessen Befestigung herangezogen werden kann. Die Laschen können auch andere als die dargestellten Formen und Lochanordnungen aufweisen.

Sind die im Einzelfall notwendigen Laschen(14 bzw. 14a bis 14e) mit ihren Zapfen(15) in die Aufnahmelöcher(5) gesteckt und dort mittels des Federrings(8) verrastet, dann wird ein Einsatz(19) in den Grundkörper(1) eingesetzt, dessen teilkugelförmige Innenkontur(20) zur Aufnahme eines nicht näher dargestellten künstlichen Kugelkopfes dient. Der Einsatz(19) besteht gewöhnlich aus Kunststoff. Er weist Noppen(21) auf, die durch die Ausschnitte(18) in die Aussparungen(9) des Grundkörpers(1) passen. Ein Ansatz(22) des Einsatzes(19) greift in die Vertiefung(10). Zur Verrastung des Einsatzes(19) in dem Grundkörper(1) ist der Einsatz(19) mit wenigstens einem Höcker(23) versehen, der in eine weitere um die Mittelachse(A) umlaufende Nut(24) des Grundkörpers(1) schnappt. Die Positionierung des Einsatzes(19) ist durch die jeweilige Anordnung der Laschen(14) nicht behindert. Der Einsatz(19) weist einen Ringrand(25) auf, der die Laschen(14) in dem Bereich, in dem sie auf der Stirnfläche(4) aufliegen, abdeckt. Der Einsatz(19) hält den Federring(8) in der Ringnut(7).

Bei der Operation wird etwa folgendermaßen vorgegangen:

Zunächst wird nach Vorbereitung des Knochens des Patienten der Grundkörper(1) implantiert. Er wird so ausgerichtet, daß seine Höhlung(2) eine geeignete Stellung hat. Auf die weiteren Befestigungsmöglichkeiten des Grundkörpers(1) braucht dabei zunächst keine Rücksicht genommen zu werden. Der Grundkörper(1) läßt sich dann durch eine in eine der Bohrungen(12) eingesetzte Befestigungsschraube(13) fixieren. Danach werden dann die für die Befestigung des Grundkörpers(1) in der Herstellung geeigneten Laschen(14 bzw. 14a bis 14d) ausgewählt und nötigenfalls vorgebogen. Die ausgewählte Lasche bzw. die ausgewählten Laschen werden danach in die der gewünschten Positionierung der Laschen entsprechenden Aufnahmelöcher(5) eingesteckt. Sie verrasten dort mittels des Federrings(8).

Anschließend wird der Einsatz(19) in die Höhlung(2) eingedrückt. Er verrastet in dieser und verhindert in seiner verrasteten Stellung, daß sich der Federring(8) so bewegen kann, daß sich die Zapfen(15) bzw. die Laschen(14) aus den Ausnahmelöchern(5) lösen können.

Schließlich werden die Laschen(14) durch eine oder mehrere ihrer Bohrungen(17) mittels Schrauben am Knochen befestigt. Dies kann auch schon vor dem Einschnappen des Einsatzes(19) geschehen.

In einer anderen Ausführung der Erfindung könnte auch der Federring(8) eingespart werden. In diesem Fall hält dann der im Grundkörper(1) durch Einschnappen oder in sonstiger Weise befestigte Einsatz(19) die Zapfen(15) direkt in den Aufnahmelöchern(5). Dies kann dadurch geschehen, daß der Einsatz(19) selbst mit einem Vorsprung in die Ringnut(7) und in die Einbuchtungen(16) der Zapfen(15) einschnappt. Dies kann jedoch auch dadurch geschehen, daß der Ringrand(25) des Einsatzes(19) die Laschen(14) an der Stirnfläche(4) hält. In diesem Fall kann außer dem Federring(8) auch die Ringnut(7) entfallen.

Die Längsachsen(L) der Aufnahmelöcher(5) könnten auch schräg zur Mittelachse(A) stehen. In diesem Fall könnten die Aufnahmelöcher(5) tiefer gestaltet sein.

Es ist nicht nötig, daß die Laschen(14) zur Befestigung am Knochen Bohrungen(17) aufweisen. Die Laschen(14) könnten auch als vorgebogene oder biegbare Haken ausgebildet sein.

Beim Ausführungsbeispiel ist der Grundkörper(1) mit der Vertiefung(10) und der Durchbrechung(11), die eine Gewindebohrung ist, versehen, damit er mittels eines Einsetzwerkzeuges in den vorbereiteten Knochen eingepreßt werden kann. Es ist jedoch statt dessen auch möglich, in an sich bekannter Weise außen am Grundkörper(1) ein in den Knochen einschraubbares Gewinde vorzusehen. Auch in diesem Falle wären die Vorteile der Erfindung erreicht.

## Patentansprüche

1. Gelenkpfanne, beispielweise für ein Hüftgelenk oder ein Schultergelenk, mit einem Bauteil(14) und einem Grundkörper (1), der einen um seine Höhlung umlaufenden Rand (3) und Aufnahmelöcher (5) aufweist, in welchen das Bauteil festlegbar ist, wobei die Aufnahmelöcher(5) im Rand(3) des Grundkörpers(1) ausgebildet sind und sich von ihrer Öffnung(6), durch die das Bauteil(14) festlegbar ist, in den Grundkörper(1) erstrecken, wobei diese Öffnungen(6) der Aufnahmelöcher(5) an der Stirnfläche(4) des Randes(3) liegen, und wobei eine Vielzahl von gleichen Aufnahmelöchern(5) im Rand(3) über 360° verteilt ist, dadurch gekennzeichnet, daß das Bauteil(14) ein die Luxation eines in die gelenkpfanne ein zusetzenden gelenkkopfes kemmendes Ringsegment oder eine Lasche(14) ist, an dem oder an der wenigstens ein Zapfen(15) befestigt ist, der durch eine der Öffnungen(6) in eines der Aufnahmelöcher(5) einsteckbar ist, und daß die Lasche (14) im eingesteckten Zustand den Rand (3) des grundkörpers (1) außen überragt.

2. Gelenkpfanne nach Anspruch 1, dadurch gekennzeichnet, daß die Aufnahmelöcher(5) und deren Öffnungen(6) am Rand(3) gleiche Abstände aufweisen.

3. Gelenkpfanne nach einem der vorhergehenden Ansprüche, dadurch gekennzeichnet, daß die Längsachsen(L) der Aufnahmelöcher(5) parallel zur Mittelachse(A) des Grundkörpers(1) verlaufen.

4. Gelenkpfanne nach einem der vorhergehenden Ansprüche, dadurch gekennzeichnet, daß an dem Bauteil(14) zwei Zapfen(15) befestigt sind, die in zwei benachbarte Aufnahmelöcher(5) einsteckbar sind, und daß der Querschnitt der Aufnahmelöcher(5) und der Zapfen(15) kreisförmig ist.

5. Gelenkpfanne nach einem der vorhergehenden Ansprüche, dadurch gekennzeichnet, daß die Lasche(14) sich radial zur Mittelachse(A) erstreckt.

6. Gelenkpfanne nach einem der vorhergehenden Ansprüche 3 und 4, dadurch gekennzeichnet, daß sich die Lasche(14) in einem spitzen Winkel zur Radialrichtung der Mittelachse(A) erstreckt.

7. Gelenkpfanne nach einem der vorhergehenden Ansprüche 3 bis 6, dadurch gekennzeichnet, daß die Lasche(14) biegbar ist.

8. Gelenkpfanne nach einem der vorhergehenden Ansprüche 3 bis 7, dadurch gekennzeichnet, daß die Lasche(14) wenigstens eine Bohrung(17) zur Aufnahme von einer Schraube aufweist.

9. Gelenkpfanne nach einem der vorhergehenden Ansprüche 3 bis 8, dadurch gekennzeichnet, daß die Aufnahmelöcher(5) sich mit einer zur Höhlung(2) offenen Ringnut(7) schneiden, in die ein Federring(8) eingesetzt ist, und daß der Federring(8) die Zapfen(15) in den Aufnahmelöchern(5) hält.

10. Gelenkpfanne nach einem der vorhergehenden Ansprüche, dadurch gekennzeichnet, daß in der Höhlung(2) ein Einsatz(19) festlegbar ist, der die Zapfen(15) in den Aufnahmelöchern(5) hält.

11. Gelenkpfanne nach Anspruch 9 und 10, dadurch gekennzeichnet, daß der Einsatz(19) in die Höhlung(2) einschnappbar ist und den Federring(8) die Zapfen(15) haltend blockiert.

12. Gelenkpfanne nach Anspruch 10, dadurch gekennzeichnet, daß der Einsatz(19) in die Ringnut(7) einrastet und dadurch die Zapfen(15) in den Aufnahmelöchern(5) hält.

13. Gelenkpfanne nach Anspruch 10 oder 11 oder 12, dadurch gekennzeichnet, daß der Einsatz(19) einen Ringrand(25) aufweist, der die Laschen(14) in den Aufnahmelöchern(5) hält.

## Claims

1. Acetabular cup, for example for a hip joint or shoulder joint, comprising a component (14) and a basic body (1), which possesses a rim (3) surrounding its cavity and seating holes (5), in which the component can be fixed, the seating holes (5) being formed in the rim (3) of the basic body (1) and extending from their mouth (6) by which the component (14) can be fixed, into the basic body (1), these mouths (6) of the seating holes (5) lying on the front face (4) of the rim (3), and a plurality of equal seating holes (5) being distributed around the rim (3) through 360°, characterized in that the component (14) is an annular segment preventing the dislocation of a joint head to be inserted into the acetabular cup or a lug (14), to which (annular segment or lug) at least one peg (15) is fixed, which can be inserted through one of the mouths (6) into one of the seating holes (5), and that the lug (14), in the inserted state, projects outwardly beyond the rim (3) of the basic body (1).

2. Acetabular cup according to Claim 1, characterized in that the seating holes (5) and their mouths (6) are equally spaced at the rim (3).

3. Acetabular cup according to one of the preceding Claims, characterized in that the longitudinal axes (L) of the seating holes (5) are parallel to the central axis (A) of the basic body (1).

4. Acetabular cup according to one of the preceding Claims, characterized in that two pegs (15) are fixed to the component (14), which pegs can be inserted into two adjacent seating holes (5), and that the cross-section of the seating holes (5) and of the pegs (15) is circular.

5. Acetabular cup according to one of the preceding Claims, characterized in that the lug (14) extends radially to the central axis (A).

6. Acetabular cup according to one of the preceding Claims 3 and 4, characterized in that the lug (14) extends at an acute angle to the radial direction of the central axis (A).

7. Acetabular cup according to one of the preceding Claims 3 to 6, characterized in that the lug (14) is flexible.

8. Acetabular cup according to one of the preceding Claims 3 to 7, characterized in that the lug (14) possesses at least one bore (17) for receiving a screw.

9. Acetabular cup according to one of the preceding Claims 3 to 8, characterized in that the seating holes (5) intersect an annular groove (7), open towards the cavity (2), into which groove a spring ring (8) is inserted, and that the spring ring (8) holds the pegs (15) in the seating holes (5).

10. Acetabular cup according to one of the preceding Claims, characterized in that an insert (19) can be secured in the cavity (2), which insert holds the pegs (15) in the seating holes (5).

11. Acetabular cup according to Claims 9 and 10, characterized in that the insert (19) can be snap-fitted into the cavity (2) and blocks the spring ring (8) as it holds the pegs (15).

12. Acetabular cup according to Claim 10, characterized in that the insert (19) engages into the annular groove (7) and thereby holds the pegs (15) in the seating holes (5).

13. Acetabular cup according to Claim 10 or 11 or 12, characterized in that the insert (19) possesses an annular flange (25), which holds the lugs (14) in the seating holes (5).

## Revendications

1. Coque acétabulaire, par exemple pour une articulation de la hanche ou pour une articulation de l'épaule comprenant un élément (14) et un corps de base (1), qui présente un bord (3) faisant le tour de sa cavité et des trous de logement (5) dans lequel l'élément peut être fixé, les trous de logements (5) étant formés dans le bord (3) du corps de base (1) et s'étendant depuis leurs orifices (6) par lesquels l'élément (14) peut être fixé, dans le corps de base (1), ces orifices (6) des trous de logement (5) se trouvant sur la surface frontale (4) du bord (3) et une pluralité de trous de logement (5) identiques étant répartis dans le bord (3) sur 360°, caractérisée en ce que l'élément (14) est un segment annulaire ou une patte (14) bloquant la luxation d'une tête d'articulation à insérer dans la coque acétabulaire, auquel ou à laquelle est fixée au moins une cheville (15) qui peut être insérée par l'une des ouvertures (6) dans l'un des trous de logement (5) et que la patte (14), l'état inséré, dépasse, à l'extérieur du bord (3), du corps de base (1).

2. Coque acétabulaire selon la revendication 1, caractérisée en ce que les trous de logement (5) et les orifices (6) présentent, au bord (3), les mêmes écartements.

3. Coque acétabulaire selon l'une des revendications précédentes, caractérisée en ce que les axes longitudinaux (L) des trous de logement (5) s'étendent parallèlement à l'axe médian (A) du corps de base (1).

4. Coque acétabulaire selon l'une des revendications précédentes, caractérisée en ce que sur l'élément (14) sont fixées deux chevilles (15) qui peuvent etre insérées dans deux trous de logement (5) voisins et que la coupe transversale des trous de logement (5) et des chevilles (15) est circulaire;

5. Coque acétabulaire selon l'une des revendications précédentes, caractérisée en ce que la patte (14) s'étend radialement par rapport à l'axe médian (A).

6. Coque acétabulaire selon l'une des revendications précédentes 3 et 4, caractérisée en ce que la patte (14) s'étend en angle aigu par rapport au sens radial de l'axe médian (A).

7. Coque acétabulaire selon l'une des revendications précédentes 3 à 6, caractérisée en ce que la patte (14) peut être arquée.

8. Coque acétabulaire selon l'une des revendications précédentes 3 à 7, caractérisée en ce que la patte (14) présente au moins un perçage (17) pour la réception d'une vis.

9. Coque acétabulaire selon l'une des revendications précédentes 3 à 8, caractérisée en ce que les trous de logement (5) sont coupés par une rainure annulaire (7) ouverte vers la cavité (2) dans laquelle est introduite une rondelle-ressort (8) et en ce que la rondelle-ressort (8) maintient les chevilles (15) dans les trous de logement (5).

10. Coque acétabulaire selon l'une des revendications précédentes, caractérisée en ce que dans la cavité (2), il peut être fixé un insert (19) qui maintient les chevilles (15) dans les trous de logement (5).

11. Coque acétabulaire selon la revendication 9 et 10, caractérisée en ce que l'insert (19) est encliquetable dans la cavité (2) et bloque la rondelle-ressort (8) en maintenant les chevilles (15).

12. Coque acétabulaire selon la revendication 10, caractérisée en ce que l'insert (19) s'encliquette dans la rainure annulaire (7) et, de ce fait, maintient les chevilles (15) dans les trous de logement (5).

13. Coque acétabulaire selon la revendication 10 ou 11 ou 12, caractérisée en ce que l'insert (19) présente un bord annulaire (25) qui maintient les pattes (14) dans les trous de logement (5).
